# EUROPÄISCHE PATENTSCHRIFT

(11) **EP 3 603 389 B1**
(45) Veröffentlichungstag und Bekanntmachung des Hinweises auf die Patenterteilung: **11.08.2021**
(21) Anmeldenummer: 19000339.2
(22) Anmeldetag: 18.07.2019
(51) Int. Cl.: A01M 7/00, G01S 13/88, G01S 15/10, G01S 15/87, G01S 15/88

(54) **LANDWIRTSCHAFTLICHE ERFASSUNGSVORRICHTUNG UND ERFASSUNGSVERFAHREN ZUR ERFASSUNG VON LANDWIRTSCHAFTLICHEN OBJEKTEN**
AGRICULTURAL DETECTION DEVICE AND METHOD FOR DETECTING AGRICULTURAL OBJECTS
DISPOSITIF DE DÉTECTION AGRICOLE ET PROCÉDÉ DE DÉTECTION PERMETTANT LA DÉTECTION DES OBJETS AGRICOLES

(30) Priorität: 03.08.2018 DE 102018006128
(43) Veröffentlichungstag der Anmeldung: 05.02.2020
(73) Patentinhaber: Pepperl+Fuchs SE, 68307 Mannheim (DE)
(72) Erfinder: Luber, Ernst, 92259 Neukirchen (DE); Seitz, Philipp, 92275 Hirschbach (DE)
(74) Vertreter: Koch Müller Patentanwaltsgesellschaft mbH

(56) Entgegenhaltungen:
- EP-A1- 2 679 085
- EP-A2- 1 978 433
- DE-A1-102011 017 621
- DE-A1-102015 111 264
- FR-A1- 3 019 969

## Beschreibung

Die Erfindung betrifft eine landwirtschaftliche Erfassungsvorrichtung und ein Erfassungsverfahren zur Erfassen von landwirtschaftlichen Objekten.

Aus der EP 2 679 085 A1 ist ein Verfahren zur Erfassung der Sprühdüsen einer Feldspritze zu den zu besprühenden Pflanzen bekannt, wobei Messfehler bzw. fehlende Messwerte ausgeglichen werden, indem anhand vorhandener Messwerte eine durchschnittliche Pflanzenhöhe bestimmt und mittels der durchschnittlichen Pflanzenhöhe ein virtueller Messwert bzw. einer Ersatzwert berechnet wird.

Vor diesem Hintergrund besteht die Aufgabe der Erfindung darin, eine Vorrichtung anzugeben, die den Stand der Technik weiterbildet.

Die Aufgabe wird durch eine landwirtschaftliche Erfassungsvorrichtung mit den Merkmalen des Patentanspruchs 1 sowie durch ein Erfassungsverfahren mit den Merkmalen des Patentanspruchs 7 gelöst. Vorteilhafte Ausgestaltungen der Erfindung sind Gegenstand von Unteransprüchen.

Gemäß einem ersten Gegenstand der Erfindung wird eine landwirtschaftliche Erfassungsvorrichtung zur Erfassung von landwirtschaftlichen Objekten, bereitgestellt, umfassend eine Sensoreinheit, eine Auswerteeinheit und eine Steuereinheit.

Die Sensoreinheit umfasst einen ersten Sensor mit einer ersten Richtcharakteristik.

Der erste Sensor ist dazu ausgelegt, ein erstes Sendesignal zu senden und ein erstes Reflexionssignal zu empfangen.

In einer ersten Ausführungsform weist der erste Sensor eine zweite Richtcharakteristik auf und sendet ein zweites Sensorsignal mit der zweiten Richtcharakteristik und empfängt ein zweites Reflexionssignal.

In einer zweiten Ausführungsform umfasst die Sensoreinheit zum Senden des zweiten Sendesignals und zum Empfangen des zweiten Reflexionssignals einen zu dem ersten Sensor benachbart angeordneten zweiten Sensor mit einer zweiten Richtcharakteristik.

Die erste Richtcharakteristik weist eine geringere Breite als die zweite Richtcharakteristik auf.

Die Steuereinheit ist eingerichtet, zwischen der ersten Richtcharakteristik und der zweiten Richtcharakteristik umzuschalten.

Die Auswerteeinheit ist dazu ausgelegt, aus dem ersten Reflexionssignal und dem zweiten Reflexionssignal die Struktur von Pflanzen und die Höhe von Pflanzen zu ermitteln.

Die Erfindung betrifft ferner Erfassungsverfahren zur Bestimmung von landwirtschaftlichen Objekten, wobei eine Sensoreinheit mit einem ersten Sensor und eine Auswerteeinheit und eine Steuereinheit vorgesehen sind.

Der erste Sensor steht in einer Wirkverbindung mit der Auswerteeinheit und der Steuereinheit.

Mittels des ersten Sensors mit einer ersten Richtcharakteristik wird in zeitlichen Abständen jeweils ein erstes Sendesignal gesendet und jeweils anschließend ein erstes Reflexionssignal empfangen.

Mittels des ersten Sensors mit einer zweiten Richtcharakteristik oder mittels eines zu dem ersten Sensor benachbart angeordneten zweiten Sensors mit einer zweiten Richtcharakteristik wird in zeitlichen Abständen jeweils ein zweites Sendesignal gesendet und jeweils anschließend ein zweites Reflexionssignal empfangen.

Die erste Richtcharakteristik weist eine geringere Breite als die zweite Richtcharakteristik auf.

Aus dem ersten Reflexionssignal und dem zweiten Reflexionssignal werden mittels der Auswerteeinheit ein Höhenwert und eine Struktur einer Pflanze bestimmt.

Mittels der Steuereinheit wird zwischen der ersten Richtcharakteristik und der zweiten Richtcharakteristik umgeschaltet. Es versteht sich, dass die Umschaltung zwischen der ersten Richtcharakteristik und der zweiten Richtcharakteristik vorzugsweise alternierend erfolgt.

Ein Vorteil ist, dass mittels der Umschaltung der Richtcharakteristik sich die Struktur der Pflanzen und der Abstand zwischen den Pflanzen schnell und zuverlässig ermitteln lassen. Insbesondere bei der Ausbringung von Wirkstoffen auf die Pflanzen ist das vorteilhaft, um die Wirkstoffmenge während des Ausbringens zu dosieren.

Vorzugsweise wird die landwirtschaftliche Erfassungsvorrichtung an landwirtschaftlichen Geräten beispielsweise an einer Feldspritze angeordnet.

Eine Feldspritze ist ein landwirtschaftliches Gerät, das Pflanzenschutzmittel oder Flüssigdünger auf landwirtschaftliche Nutzflächen gleichmäßig verteilen kann.

Bei Feldspritzen wird die Spritzbrühe durch eine dem Vorratsbehälter nachgelagerte Flüssigkeits-Pumpe unter Druck gebracht und dann mittels Spritzdüsen verteilt. Die Spritzdüsen sind typischerweise entlang eines Gestänges quer zu einer Bewegungsrichtung angeordnet.

Die erfindungsgemäße Erfassungsvorrichtung bzw. das entsprechende Erfassungsverfahren sind dazu ausgelegt, einen Höhenwert zu ermitteln. Der Höhenwert umfasst Insbesondere den Abstand der Sensoreinheit zu dem Boden oder zu einer Pflanzen oder Teile einer Pflanze.

Die erfindungsgemäße Erfassungsvorrichtung oder zumindest die Sensoreinheit der Erfassungsvorrichtung Ist hierfür beispielsweise an dem Gestänge der Feldspritze zu den Düsen benachbart angeordnet, so dass sie mit der Feldspritze mitbewegt wird und denselben Abstand zu den Pflanzen bzw. dem Boden aufweist.

Es versteht sich, dass die Abstandsregelvorrichtung einen Abstand zu einem Objekt, z.B. der behandelnden Fläche bzw. einer strukturierten Oberfläche, nämlich den Pflanzen auf einem Feld, mittels der Laufzeit eines ausgesandten und an dem Objekt bzw. einer Oberfläche des Objekts reflektierten Signals ermittelt. Es wird also die Zeit zwischen dem Senden eines Sendesignals, z.B. eines Ultraschallpulses, und dem anschließenden Empfangen des an Oberflächen reflektierten Sendesignals, welches hier als Reflexionssignal bezeichnet wird, gemessen.

Insbesondere werden Abstände mittels zweier unterschiedlicher Richtcharakteristiken erfasst. Die Richtcharakteristik eines Sensors beschreibt die Winkelabhängigkeit der Stärke empfangener oder gesendeter Signale.

Im Fall von Schallsensoren, z.B. Ultraschallwandlern, spricht man typischerweise von einer Schallkeule. Für Radarsensoren wird üblicherweise von einem Erfassungsfeld gesprochen.

Unterschiedliche Richtcharakteristiken können entsprechend durch gleichartige, aber unterschiedlich konfiguriert oder betriebene Sensoren oder auch durch unterschiedliche Sensortypen, also Sensoren mit unterschiedlichen physikalischen Wirkprinzipien realisiert werden.

Die Kombination einer schmalen und einer breiten Richtcharakteristik ermöglicht es, auch bei einer geringen Pflanzendichte noch zuverlässig den Abstand zu ermitteln. Darüber hinaus ermöglicht die Kombination auch das Erfassen von horizontalen Abständen zwischen den einzelnen Pflanzen sowie das gleichzeitige zuverlässige Erfassen der Pflanzenspitze einerseits und des Bodens andererseits.

So können zusätzlich die Höhe der Pflanzen sowie die Dichte der Pflanzen bestimmt und hieraus die Pflanzenmasse werden. Hierdurch lässt sich insbesondere, die Menge des Düngemittelauftrags an den wirklichen Bedarf der Pflanzen anzupassen und den zu erwartenden Ernteertrag abzuschätzen und Kosten sparen. Des Weiteren lässt sich die Umweltbelastung verringern.

Es versteht sich, dass die Kombination der Messergebnisse darauf beruht, dass diese möglichst an bzw. von demselben Ort erfasst werden, weshalb die erste und zweite Sensoreinheit zueinander benachbart angeordnet sind. Bevorzugt, ist der Abstand zwischen den Sensoren so klein wie möglich.

Der Abstand von der Mitte einer Sendesignalquelle des ersten Sensors zu der Mitte einer Sendesignalquelle des zweiten Sensors beträgt bevorzugt höchstens 10 cm oder höchstens 5 cm oder höchstens 1 cm.

Gemäß einer ersten Ausführungsform ist der erste Sensor ein Ultraschallsensor. Die erste Richtcharakteristik ist bevorzugt eine Schallkeule, z.B. eines Ultraschallwandlers, wobei die Schallkeule bevorzugt in einem Abstand von 1m zu dem ersten Sensor eine Breite zwischen 30 cm und 70 cm aufweist.

Gemäß einer weiteren Ausführungsform ist die zweite Richtcharakteristik eine Schallkeule und die Schallkeule weist in einem Abstand von 1m zu dem ersten Sensor eine Breite zwischen 80 cm und 120 cm auf.

Die zweite Schallkeule wird bevorzugt mittels eines zweiten Sensors oder alternativ mittels des ersten Sensors realisiert, wobei der erste Sensor hierfür besonders bevorzugt als Ultraschallwandler mit veränderbarer Richtcharakteristik ausgebildet ist.

In einer alternativen Ausführungsform ist der zweite Sensor ein Radarsensor mit einem Erfassungsfeld als zweite Richtcharakteristik, wobei das Erfassungsfeld in einem Abstand von 1m zu dem Radarsensor eine Breite zwischen 80cm und 2m aufweist.

In einer weiteren Ausführungsform des Erfassungsverfahrens wird das zweite Sendesignal mittels eines zweiten Sensors gesendet, wobei jeweils ein erstes und ein zweites Sendesignal gleichzeitig oder zumindest im Wesentlichen gleichzeitig gesendet werden.

In einer alternativen Ausführungsform werden die ersten Sendesignale und die zweiten Sendesignale alternierend gesendet. Es versteht sich, dass die zweiten Sendesignale in diesem Fall von dem ersten Sensor mit entsprechend veränderbarer Richtcharakteristik oder von einem zweiten Sensor erzeugt und empfangen werden können.

Gemäß einer Weiterbildung werden die ersten Sendesignale mittels eines ersten Ultraschallwandlers erzeugt.

Die zweiten Sendesignale werden gemäß zweier alternativen Ausführungsformen mittels eines zweiten Ultraschallwandlers oder mittels eines Radarsensors erzeugt.

Gemäß einer weiteren Ausführungsform wird in den Reflexionssignalen eine aufsteigende Flanke mittels eines Steigungsschwellwerts ermittelt.

In einer anderen Ausführungsform wird in den Reflexionssignalen ein Höhenwert mittels eines Höhenschwellwerts ermittelt. Die Schwellwertvergleiche werden besonders bevorzugt mittels digitaler Schwellwertfilter realisiert.

Es versteht sich, dass eine Reflexion des Sendesignals an einer Oberfläche einen Peak bzw. verschiedene Reflexionen aus verschiedenen Reflexionsebenen der Pflanze bis zu dem Boden unterschiedliche bzw. mehrere Peaks in dem Reflexionssignal verursachen.

Die einzelnen Peaks können sowohl anhand des Anstiegs, also der ansteigenden Flanke bzw. einer Zunahme der Steigung des Signals als auch an den besonders hohen Signalwerten im Bereich der Peak-Spitze erkannt werden.

Gemäß einer Weiterbildung wird ein Höhenwert nach Überschreiten eines Steigungsschwellenwerts und eines Höhenschwellwerts ermittelt wird. Mittels des Steigungsschwellwerts wird der Beginn einer aufsteigenden Flanke ermittelt.

Ein nachfolgendes Unterschreiten der Steigungsschwellwerts bedeutet das Ende der Flanke. Überschreitet die Höhe der Flanke zusätzlich einen Höhenschwellwert, so wird die Flanke als Reflexion gewertet. Als Beginn der Reflexion wird beispielsweise der Zeitpunkt des Flankenanstiegs, also der Zeitpunkt des Überschreitens des Steigungsschwellwerts gewertet.

Die Kombination der beiden Schwellwertfilter ermöglicht eine besonders zuverlässige Auswertung der Reflexionssignale. Insbesondere sind einzelne Reflexionsebenen einer Pflanze genauer detektierbar.

In einer weiteren Weiterbildung werden die Höhenwerte mittels eines dynamischen Schwellenwertverfahren ermittelt.

Bevorzugt umfasst die Auswerteeinheit zur Umsetzung einer oder mehrerer Schwellwertabfragen entsprechende digitale Filter in Form von Logikbausteinen oder in Form eines Signalprozessors mit einem Programm.

In einer weiteren Ausführungsform ist die Auswerteeinheit dazu ausgelegt, aus dem Höhenwert die Höhe des landwirtschaftlichen Geräts zu regeln. Der Höhenwert umfasst hierbei bei dem landwirtschaftlichen Gerät einen Abstand des Gestänges bzw. der Spritzdüsen zu einer zu behandelnden Fläche, z.B. dem Boden und/oder Pflanzen bzw. Pflanzenspitzen. Ein gleichmäßiger Abstand zwischen den Spritzdüsen und den zu behandelnden Pflanzen ermöglicht eine zuverlässige und Ressourcen sparende Behandlung.

Mittels des Höhenwertes ist es möglich während des Betriebs insbesondere den Abstand der Düsen möglichst schnell an eine Änderung der Pflanzenhöhe anzupassen d.h. zu regeln.

Die Erfindung wird nachfolgend unter Bezugnahme auf die Zeichnungen näher erläutert. Hierbei werden gleichartige Teile mit identischen Bezeichnungen beschriftet. Die dargestellten Ausführungsformen sind stark schematisiert, d.h. die Abstände und die lateralen und die vertikalen Erstreckungen sind nicht maßstäblich und weisen, sofern nicht anders angegeben, auch keine ableitbaren geometrischen Relationen zueinander auf. Darin zeigen, die
- Figur 1: eine Seitenansicht auf eine erste erfindungsgemäße Anordnung einer erfindungsgemäßen Erfassungsvorrichtung an einer Feldspritze,
- Figur 2: eine Rückansicht der Feldspritze aus Figur 1,
- Figur 3: eine Ansicht auf eine erste erfindungsgemäße Ausführungsform einer Erfassungsvorrichtung,
- Figur 4: eine Ansicht eines ersten Reflexionssignals,
- Figur 5: eine Ansicht von Schallkeulen eines Ultraschallsensors für unterschiedliche Sendeleistungen und
- Figur 6: eine Ansicht von Schallkeulen unterschiedlicher Ultraschallsensoren.

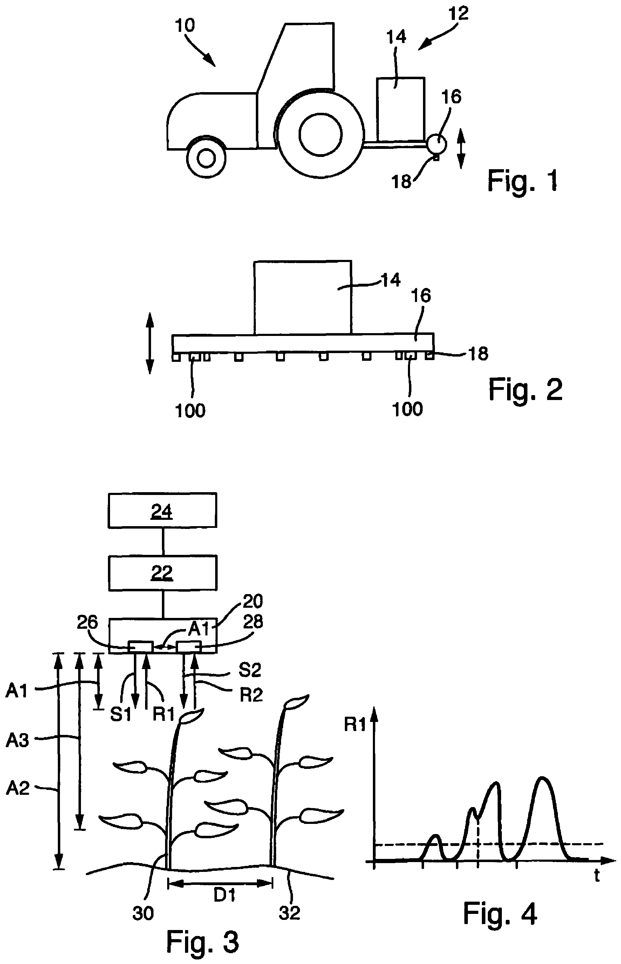

Die Abbildungen der Figuren 1 und 2 zeigen einen Traktor 10 mit einer Feldspritze 12 in einer seitlichen Ansicht bzw. einer Rückansicht. Die Feldspritze 12 umfasst einen Tank 14 für eine Spritzbrühe sowie einem Spritzgestänge 16. Entlang des Spritzgestänges 16 sind Spritzdüsen 18 angeordnet.

Das Spritzgestänge 16 der Feldspritze 12 Ist in einer Ausführungsform in der Höhe fixiert und in einer alternativen Ausführungsform In vertikaler Richtung beweglich.

Um den Abstand der Spritzdüsen 18 zu dem Boden und/oder den Pflanzen bzw. unterschiedlichen Pflanzenebenen sowie die Größe der Pflanzen und die Dichte des Pflanzenwuchses zu bestimmen, ist an dem Spritzgestänge 16 zu mindestens einer Spritzdüsen 18 benachbart zumindest ein Teil einer erfindungsgemäßen Erfassungsvorrichtung 100 angeordnet.

In der Abbildung der Figur 3 ist eine erste Ausführungsform einer erfindungsgemäßen Erfassungsvorrichtung 100 dargestellt. Im Folgenden werden nur die Unterschiede zu der Abbildung der Figur 1 erläutert.

Die Erfassungsvorrichtung 100 weist eine Sensoreinheit 20, eine Auswerteeinheit 22 und eine Steuereinheit 24 auf. Die Sensoreinheit 20 umfasst einen ersten Sensor 26 und einen in einem Abstand A1 zu dem ersten Sensor 28 angeordneten zweiten Sensor 28.

Der erste Sensor 26 und der zweite Sensor 28 unterscheiden sich hinsichtlich ihrer Richtcharakteristik. Der erste Sensor weist die erste Richtcharakteristik auf und der zweite Sensor weist die zweite Richtcharakteristik auf, wobei die Richtcharakteristik eine schmalere Breite aufweist als die zweite Richtcharakteristik.

Werden die beiden Sensoren 26 und 28 alternierend betrieben, so sendet zu einem ersten Zeitpunkt t1 der erste Sensor 26 ein erstes Sendesignal S1 aus. Das gesendete Sendesignale S1 wird an den verschiedenen Ebenen einer darunter stehenden Pflanze 30, beispielsweise der Pflanzenspitze und/oder einem oder mehreren Blättern der Pflanze, und/oder von einem darunter liegenden Boden reflektiert. Die Reflexionen werden als erstes Reflexionssignal R1 von dem ersten Sensor 26 empfangen und an die Auswerteeinheit 22 übermittelt.

Anschließend zu einem zweiten Zeitpunkt t2 = t1+Dt sendet der zweite Sensor 28 ein zweites Sendesignal 28 aus und erfasst die Reflexionen als ein zweites Reflexionssignal R2, welches ebenfalls an die Auswerteeinheit 22 übermittelt wird.

Mittels der Auswerteeinheit 22 werden anschließend anhand der Reflexionssignale R1 und R2 der Abstand A1 der Spitze zu der Sensoreinheit 20, der Abstand A2 des Bodens sowie der Abstand A3 dazwischenliegender Reflexionsebenen, z.B. Blätter, bestimmt.

Außerdem wird anhand der Reflexionssignale R1 und R2 ein horizontaler Abstand D1 zwischen benachbarten Pflanzen bestimmt.

Anhand der bestimmten Abstände wird mittels der Steuerungseinheit 24 der Abstand der Spritzdüsen zu den Pflanzen eingestellt. Außerdem ermöglichen es die ermittelten Abstände, die abzugebende Menge an Düngemittel an die Pflanzenhöhe und/oder die Pflanzendichte anzupassen.

Wird die Erfassungsvorrichtung über ein ganzes Feld bewegt und die Abstände A1, A2 und A3 sowie die horizontalen Abstände D1 in regelmäßigen zeitlichen Abständen erfasst, so ist es außerdem möglich sowohl eine durchschnittliche Pflanzenhöhe als auch eine durchschnittliche Pflanzendichte für das gesamte Feld zu bestimmen, wodurch ein zu erwartender Ertrag abschätzbar ist.

In der Abbildung der Figur 4 ist ein Beispiel für ein Reflexionssignal R1 über der Zeit t aufgetragen. Jede Reflexion, also jede Reflexionsebene erzeugt einen Peak in dem Reflexionssignal.

Im Idealfall wird ein erster Peak durch die Pflanzenspitze erzeugt und ein letzter Peak durch den Boden und gegebenenfalls weitere dazwischen liegende Peaks durch weitere Reflexionsebenen der Pflanze, also beispielsweise Blätter.

Die Peaks, also die einzelnen Reflexionen können allerdings auch verschmelzen bzw. verschiedene Reflektionen oder auch Streuung können sich überlagern.

Die Peaks werden mittels Schwellwertabfragen bzw. mittels eines oder mehrerer Schwellwertfilter ermittelt. Um die einzelnen Reflexionsebenen einer Pflanze genauer zu detektieren ist es vorteilhaft, eine Kombinierte Abfrage eines Steigungsschwellwerts und eines Höhenschwellwerts durchzuführen bzw. entsprechende Filter, z.B. digitale Filter, anzuwenden.

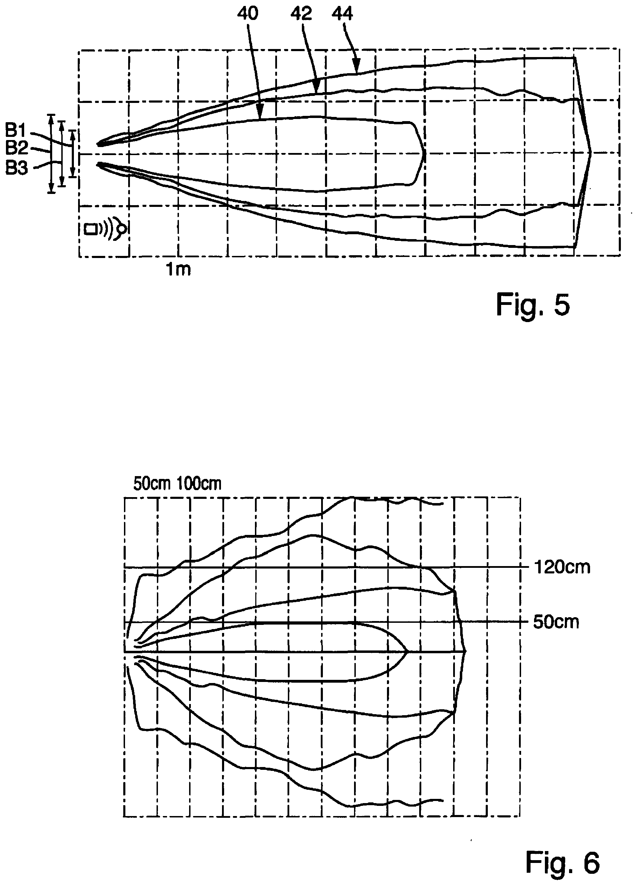

In der Abbildung der Figur 5 sind die Schallkeulen 40, 42, 44 eines Ultraschallsensors mit veränderbarer Sendeleistung für drei unterschiedliche Sendeleistungen dargestellt.

Die Schallkeulen 40, 42 und 44 und damit die Richtcharakteristiken für die unterschiedlichen Sendeleistungen weisen jeweils unterschiedliche Breiten B1, B2 bzw. B3 auf. Dies ermöglicht es, die beiden erfindungsgemäß unterschiedlichen Richtcharakteristiken mit dem ersten Sensor und ohne einen zweiten Sensor zu realisieren.

Alternativ ist es möglich zwei unterschiedliche Sensoren, z.B. zwei unterschiedliche Ultraschallsensoren, einzusetzen, die sich voneinander zumindest hinsichtlich der Richtcharakteristik unterscheiden. In der Abbildung der Figur 6 sind die Schallkeulen von vier unterschiedlichen Ultraschallsensoren dargestellt, die sich alle insbesondere hinsichtlich Ihrer Breite deutlich unterscheiden.

## Patentansprüche

1. Landwirtschaftliche Erfassungsvorrichtung, umfassend eine Sensoreinheit, eine Auswerteeinheit und eine Steuereinheit, wobei
- die Sensoreinheit einen ersten Sensor mit einer ersten Richtcharakteristik umfasst,
- der erste Sensor dazu ausgelegt ist ein erstes Sendesignal zu senden und ein erstes Reflexionssignal zu empfangen,
**dadurch gekennzeichnet, dass**
in einer ersten Ausführungsform der erste Sensor eine zweite Richtcharakteristik aufweist und ein zweites Sensorsignal mit der zweiten Richtcharakteristik sendet und eine zweites Reflexionssignal empfängt oder in einer zweiten Ausführungsform die Sensoreinheit zum Senden des zweiten Sendesignals und zum Empfangen des zweiten Reflexionssignals einen zu dem ersten Sensor benachbart angeordneten zweiten Sensor mit einer zweiten Richtcharakteristik umfasst, wobei die erste Richtcharakteristik eine geringere Breite als die zweite Richtcharakteristik aufweist und die Steuereinheit eingerichtet ist, zwischen der ersten Richtcharakteristik und der zweiten Richtcharakteristik umzuschalten und die Auswerteeinheit dazu ausgelegt ist, aus dem ersten Reflexionssignal und aus dem zweiten Reflexionssignal wenigstens die Struktur von Pflanzen und ein Höhenwert zu ermitteln.

2. Landwirtschaftliche Erfassungsvorrichtung nach Anspruch 1, **dadurch gekennzeichnet, dass** der erste Sensor ein Ultraschallsensor ist.

3. Landwirtschaftliche Erfassungsvorrichtung nach Anspruch 1 oder 2, **dadurch gekennzeichnet, dass** die erste Richtcharakteristik eine Schallkeule ist und die Schallkeule in einem Abstand von 1m zu dem ersten Sensor eine Breite zwischen 30 cm und 70 cm aufweist.

4. Landwirtschaftliche Erfassungsvorrichtung nach einem der vorangegangenen Ansprüche, **dadurch gekennzeichnet, dass** die zweite Richtcharakteristik eine Schallkeule ist und die Schallkeule in einem Abstand von 1m zu dem ersten Sensor eine Breite zwischen 80 cm und 120 cm aufweist.

5. Landwirtschaftliche Erfassungsvorrichtung nach einem der vorangegangenen Ansprüche, **dadurch gekennzeichnet, dass** der zweite Sensor ein Radarsensor mit einem Erfassungsfeld als zweite Richtcharakteristik Ist, wobei das Erfassungsfeld in einem Abstand von 1 m zu dem Radarsensor eine Breite zwischen 80 cm und 2 m aufweist.

6. Landwirtschaftliche Erfassungsvorrichtung nach einem der vorangegangenen Ansprüche, **dadurch gekennzeichnet, dass** der erste Sensor als Ultraschallwandler mit einer veränderbarer Richtcharakteristik ausgebildet ist.

7. Erfassungsverfahren zur Bestimmung von landwirtschaftlichen Objekten, wobei
eine Sensoreinheit mit einem ersten Sensor und eine Auswerteeinheit und eine Steuereinheit vorgesehen ist, und der erste Sensor in einer Wirkverbindung mit der Auswerteeinheit und der Steuereinheit steht,
- mittels des ersten Sensors mit einer ersten Richtcharakteristik in zeitlichen Abständen jeweils ein erstes Sendesignal gesendet und jeweils anschließend ein erstes Reflexionssignal empfangen wird,
**dadurch gekennzeichnet, dass**
mittels des ersten Sensors mit einer zweiten Richtcharakteristik oder mittels eines zu dem ersten Sensor benachbart angeordneten zweiten Sensors mit einer zweiten Richtcharakteristik in zeitlichen Abständen jeweils ein zweites Sendesignal gesendet und jeweils anschließend ein zweites Reflexionssignal empfangen wird, wobei
- die erste Richtcharakteristik eine geringere Breite als die zweite Richtcharakteristik aufweist und
- aus dem ersten Reflexionssignal und dem zweiten Reflexionssignal ein Höhenwert und eine Struktur einer Pflanze bestimmt wird.

8. Erfassungsverfahren nach Anspruch 7, **dadurch gekennzeichnet, dass** das zweite Sendesignal mittels eines zweiten Sensors gesendet wird, wobei jeweils ein erstes und ein zweites Sendesignal gleichzeitig oder zumindest im Wesentlichen gleichzeitig gesendet werden.

9. Erfassungsverfahren nach Anspruch 7, **dadurch gekennzeichnet, dass** die ersten Sendesignale und die zweiten Sendesignale alternierend gesendet werden.

10. Erfassungsverfahren nach einem der Ansprüche 7 bis 9, **dadurch gekennzeichnet, dass** die ersten Sendesignale mittels eines ersten Ultraschallwandlers erzeugt werden.

11. Erfassungsverfahren nach einem der Ansprüche 7 bis 10, **dadurch gekennzeichnet, dass** die zweiten Sendesignale mittels eines zweiten Ultraschallwandlers oder mittels eines Radarsensors erzeugt werden.

12. Erfassungsverfahren nach einem der Ansprüche 7 bis 11, **dadurch gekennzeichnet, dass** in den Reflexionssignalen eine aufsteigende Flanke mittels eines Steigungsschwellwerts ermittelt wird.

13. Erfassungsverfahren nach einem der Ansprüche 7 bis 12, **dadurch gekennzeichnet, dass** in den Reflexionssignalen ein Höhenwert mittels eines Höhenschwellwerts ermittelt wird.

14. Erfassungsverfahren nach einem der Ansprüche 7 bis 13, **dadurch gekennzeichnet, dass** ein Höhenwert nach Überschreiten eines Steigungsschwellwerts und eines Höhenschwellwerts ermittelt wird.

## Claims

1. Agricultural detection device comprising a sensor unit, an evaluating unit and a control unit, wherein
- the sensor unit comprises a first sensor with a first directional characteristic,
- the first sensor is configured for the purpose of transmitting a first transmission signal and receiving a first reflection signal,
**characterised in that**
in a first form of embodiment the first sensor has a second directional characteristic and transmits a second sensor signal with the second directional characteristic and receives a second reflection signal or in a second form of embodiment the sensor unit comprises a second sensor, which is arranged adjacent to the first sensor, with a second directional characteristic for transmission of the second transmission signal and for reception of the second reflection signal, wherein the first directional characteristic has a smaller width than the second directional characteristic and the control unit is arranged to switch over between the first directional characteristic and the second directional characteristic and the evaluating unit is configured for the purpose of determining at least the structure of plants and a height value from the first reflection signal and from the second reflection signal.

2. Agricultural detection device according to claim 1, **characterised in that** the first sensor is an ultrasound sensor.

3. Agricultural detection device according to claim 1 or 2, **characterised in that** the first directional characteristic is a sonic beam and the sonic beam at a spacing of 1 metre from the first sensor has a width between 30 centimetres and 70 centimetres.

4. Agricultural detection device according to any one of the preceding claims, **characterised in that** the second directional characteristic is a sonic beam and the sonic beam at a spacing of 1 metre from the first sensor has a width between 80 centimetres and 120 centimetres.

5. Agricultural detection device according to any one of the preceding claims, **characterised in that** the second sensor is a radar sensor with a detection field as second directional characteristic, wherein the detection field at a spacing of 1 metre from the radar sensor has a width between 80 centimetres and 2 metres.

6. Agricultural detection device according to any one of the preceding claims, **characterised in that** the first sensor is constructed as an ultrasound transducer with a variable directional characteristic.

7. Detection method for determining agricultural objects, wherein
a sensor unit with a first sensor and an evaluating unit and a control unit is provided, and the first sensor is in operative connection with the evaluating unit and the control unit,
- by means of the first sensor with a first directional characteristic a respective first transmission signal is transmitted at intervals in time and subsequently a respective first reflection signal is received,
**characterised in that**
by means of the first sensor with a second directional characteristic or by means of a second sensor, which is arranged adjacent to the first sensor, with a second directional characteristic a respective second transmission signal is transmitted at intervals in time and subsequently a respective second reflection signal is received, wherein
- the first directional characteristic has a smaller width than the second directional characteristic and
- a height value and a structure of a plant are determined from the first reflection signal and the second reflection signal.

8. Detection method according to claim 7, **characterised in that** the second transmission signal is transmitted by means of a second sensor, wherein a respective first and second transmission signal are transmitted simultaneously or at least substantially simultaneously.

9. Detection method according to claim 7, **characterised in that** the first transmission signals and the second transmission signals are transmitted in alternation.

10. Detection method according to any one of claims 7 to 9, **characterised in that** the first transmission signals are generated by means of a first ultrasound transducer.

11. Detection method according to any one of claims 7 to 10, **characterised in that** the second transmission signals are generated by means of a second ultrasound transducer or by means of a radar sensor.

12. Detection method according to any one of claims 7 to 11, **characterised in that** a rising flank is determined in the reflection signals by means of a gradient threshold value.

13. Detection method according to any one of claims 7 to 12, **characterised in that** a height value is determined in the reflection signals by means of a height threshold value.

14. Detection method according to any one of claims 7 to 13, **characterised in that** a height value is ascertained after exceeding of a gradient threshold value and a height threshold value.

## Revendications

1. Dispositif de détection agricole comprenant une unité de capteur, une unité d'analyse et une unité de commande, dans lequel
- l'unité de capteur comprend un premier capteur avec une première caractéristique directionnelle,
- le premier capteur est conçu pour émettre un premier signal d'émission et pour recevoir u premier signal de réflexion,
**caractérisé en ce que**,
dans une première forme de réalisation, le premier capteur présente une deuxième caractéristique directionnelle et émet un deuxième signal de capteur avec la deuxième caractéristique directionnelle et reçoit un deuxième signal de réflexion ou, dans une deuxième forme de réalisation, l'unité de capteur comprend un deuxième capteur avec une deuxième caractéristique directionnelle disposé à proximité du premier capteur pour émettre le deuxième signal d'émission et pour recevoir le deuxième signal de réflexion, la première caractéristique directionnelle présentant une largeur plus faible que la deuxième caractéristique directionnelle et l'unité de commande étant agencée pour basculer entre la première caractéristique directionnelle et la deuxième caractéristique directionnelle et l'unité d'analyse étant conçue pour déterminer à partir du premier signal de réflexion et à partir du deuxième de réflexion au moins la structure de plantes et une valeur de hauteur.

2. Dispositif de détection agricole selon la revendication 1, **caractérisé en ce que** le premier capteur est un capteur d'ultrasons.

3. Dispositif de détection agricole selon la revendication 1 ou 2, **caractérisé en ce que** la première caractéristique directionnelle est un lobe acoustique et que le lobe acoustique présente, à une distance de 1 m du premier capteur, une largeur de 30 cm à 70 cm.

4. Dispositif de détection agricole selon l'une des revendications précédentes, **caractérisé en ce que** la deuxième caractéristique directionnelle est un lobe acoustique et que le lobe acoustique présente, à une distance de 1 m du premier capteur, une largeur de 80 cm à 120 cm.

5. Dispositif de détection agricole selon l'une des revendications précédentes, **caractérisé en ce que** le deuxième capteur est un capteur radar avec un champ de détection comme deuxième caractéristique directionnelle, le champ de détection présentant, à une distance de 1 m du capteur radar, une largeur de 80 cm à 2 m.

6. Dispositif de détection agricole selon l'une des revendications précédentes, **caractérisé en ce que** le premier capteur est configuré comme un convertisseur a ultrasons avec une caractéristique directionnelle variable.

7. Procédé de détection pour déterminer des objets agricoles, selon lequel
il est prévu une unité de capteur avec un premier capteur et une unité d'analyse et une unité de commande, et le premier capteur est en liaison active avec l'unité d'analyse et l'unité de commande, un premier signal d'émission étant émis à intervalles temporels au moyen du premier capteur avec la première caractéristique directionnelle et un premier signal de réflexion étant ensuite reçu,
**caractérisé en ce que**,
au moyen du premier capteur avec une deuxième caractéristique directionnelle ou au moyen d'un deuxième capteur avec une deuxième caractéristique directionnelle disposé à proximité du premier capteur, un deuxième signal d'émission est émis à intervalles temporels et un deuxième signal de réflexion est ensuite reçu,
- la première caractéristique directionnelle présentant une largeur plus faible que la deuxième caractéristique directionnelle et
- une valeur de hauteur et une structure d'une plante étant déterminées à partir du premier signal de réflexion et du deuxième signal de réflexion.

8. Procédé de détection selon la revendication 7, **caractérisé en ce que** le deuxième signal d'émission est émis au moyen d'un deuxième capteur, un premier et un deuxième signal d'émission étant émis simultanément ou au moins sensiblement simultanément.

9. Procédé de détection selon la revendication 7, **caractérisé en ce que** les premiers signaux d'émission et les deuxièmes signaux d'émission sont émis alternativement.

10. Procédé de détection selon l'une des revendications 7 à 9, **caractérisé en ce que** les premiers signaux d'émission sont générés au moyen d'un premier convertisseur à ultrasons.

11. Procédé de détection selon l'une des revendications 7 à 10, **caractérisé en ce que** les deuxièmes signaux d'émission sont générés au moyen d'un deuxième convertisseur à ultrasons ou au moyen d'un capteur radar.

12. Procédé de détection selon l'une des revendications 7 à 11, **caractérisé en ce que**, dans les signaux de réflexion, un flanc montant est déterminé au moyen d'une valeur seuil de montée.

13. Procédé de détection selon l'une des revendications 7 à 12, **caractérisé en ce que**, dans les signaux de réflexion, une valeur de hauteur est déterminée au moyen d'une valeur seuil de hauteur.

14. Procédé de détection selon l'une des revendications 7 à 13, **caractérisé en ce qu'**une valeur de hauteur est déterminée après le dépassement d'une valeur seuil de montée et d'une valeur seuil de hauteur.
